# EUROPEAN PATENT APPLICATION

(11) **EP 4 614 158 A1**
(43) Date of publication of application: **10.09.2025**
(21) Application number: 23885503.5
(22) Date of filing: 13.10.2023
(51) Int. Cl.: G01N 35/02, B03C 1/00, B03C 1/01, B03C 1/24, B03C 1/28, G01N 33/543, G01N 33/553

(54) **MAGNETIC COLLECTING UNIT, AND TESTING DEVICE**

(30) Priority: 31.10.2022 JP 2022175125
(71) Applicant: FUJIFILM Corporation, Tokyo 106-8620 (JP)
(72) Inventor: HIBE, Daisuke, Ashigarakami-gun, Kanagawa 258-8538 (JP)
(74) Representative: Dehns Germany Partnerschaft mbB
(86) International application number: PCT/JP2023/037295
(87) International publication number: WO 2024/095741

(57) **Abstract**

A magnetic collection unit generates a magnetic field in an inside of a reaction cell accommodating a suspension containing the magnetic particles and magnetically collects the magnetic particles in the suspension on an inner wall surface of the reaction cell. The magnetic collection unit includes a magnetic field generation unit that includes two magnets having a length equal to or longer than a distance from a liquid surface of the suspension in the reaction cell to a bottom surface of the reaction cell and generating a magnetic field across a range from the liquid surface to the bottom surface and includes a non-magnetic body, where the two magnets are disposed such that surfaces not having a magnetic pole face each other with the non-magnetic body being sandwiched therebetween, and the two magnets are disposed such that magnetic poles different from each other face the reaction cell; and a moving mechanism that moves the magnetic field generation unit between a magnetic collection position at which an upper end of each of the magnets is positioned on the liquid surface or above the liquid surface and a retreat position at which the magnetic field does not affect the reaction cell.

## Description

### BACKGROUND OF THE INVENTION

### 1. Field of the Invention

The present disclosure relates to a magnetic collection unit and an examination device.

### 2. Description of the Related Art

An examination device that quantitatively or qualitatively detects an examination target substance in a specimen has been known. Many of such examination devices use an immunoassay principle, and examples thereof include a chemiluminescent enzyme immunological analysis device and a fluorescence immunological analysis device (for example, JP2016-085093A).

Such an examination device carries out a detection treatment of detecting an examination target substance in a specimen by detecting luminescence or fluorescence based on a labeling substance such as an enzyme label or a fluorescent label attached to the examination target substance in the specimen by using an immunoreaction. In addition, before such a detection treatment of the examination target substance, a pretreatment such as attaching a labeling substance to the examination target substance in the specimen is carried out on the specimen. In some cases, the examination device is configured to automatically execute the pretreatment and the detection treatment and output the detection result in a case where the pretreatment and the detection treatment are automated and a specimen collection container accommodating the collected specimen is loaded.

In such an automated examination device, in order to attach a labeling substance to an examination target substance in a specimen, for example, such a treatment as described later is carried out using magnetic particles as a solid phase. First, in a reaction cell, magnetic particles modified with a first binding substance (for example, a primary antibody) that specifically binds to the target substance (for example, an antigen) are mixed with the specimen, and thus the target substance and the first binding substance are bound to each other to generate an immune complex. As a result, the target substance is captured by the magnetic particles through the first binding substance. Thereafter, the generated immune complex is separated from the immune complex and a component derived from the specimen (unreacted substance) which does not form the immune complex, that is, so-called bound/free (B/F) separation is carried out. In a case of the B/F separation, the liquid is suctioned in a state in which the magnetic particles are temporarily adsorbed to an inner wall surface of the reaction cell by a magnet disposed outside the reaction cell. Thereafter, a washing solution is discharged to the reaction cell, and the mixed liquid is suctioned and discharged in a state in which the washing solution and the magnetic particles are mixed, whereby the magnetic particles are washed. Next, a labeling reagent containing a second binding substance (for example, a secondary antibody) that specifically binds to the target substance is mixed with the magnetic particles, where the second binding substance has been bound to the labeling substance. As a result, the target substance captured on the magnetic particles through the first binding substance and the second binding substance is bound to each other, and thus a sandwich type immune complex in which the target substance is sandwiched between the first binding substance and the second binding substance is generated. Thereafter, the magnetic particles are cleaned again by mixing the washing solution and the magnetic particles for the B/F separation. In a case where the label is an enzyme label, the magnetic particles and a reagent containing a luminescent substrate are further mixed and subjected to the detection treatment.

JP2006-218442A proposes a magnetic collection device (magnetic collection unit) that obtains a high gradient magnetic field, where the magnetic collection device is for shortening the time required for adsorbing magnetic particles to an inner wall surface during B/F separation. The magnetic collection device according to JP2006-218442A is a pair of magnets that are obtained by disposing the same poles repelling each other to face each other, where a separation container (reaction cell) is disposed to be brought close to a gap between the magnetic poles to increase the magnetic field strength that is generated in a reaction cell.

### SUMMARY OF THE INVENTION

By providing the pair of magnets described in JP2006-218442A, it is possible to magnetically collect the magnetic particles on the inner surface of the side wall surface of the reaction cell that faces the gap between the pair of magnetic poles in a short time. In the case of JP2006-218442A, the magnetic field intensity is maximized on the side wall surface of the reaction cell that faces the gap between the pair of magnets, and thus the magnetic particles are concentrated at a place facing the gap.

In a case where the magnetic particles are concentrated and magnetically collected in a part of a region of the side wall surface of the reaction cell, a lump of the magnetically collected magnetic particles is formed to have a certain thickness from the side wall surface. In the reaction cell, after the magnetic particles are magnetically collected, a treatment of inserting a nozzle and suctioning a liquid in the reaction cell is carried out. In a case where the lump of the magnetic particles is thick, the distal end of the nozzle may touch the lump of the magnetic particles in a case where the nozzle is inserted into the reaction cell, and a part of the magnetic particles may be suctioned together with the liquid during the liquid suction. The loss of the magnetic particles due to the suction of the magnetic particles by the nozzle in the washing leads to a decrease in measurement accuracy in a case of carrying out a quantitative measurement of an examination target substance.

The present disclosure has been made in consideration of the above circumstances, and an object of the present disclosure is to provide a magnetic collection unit that makes it possible to collect magnetic particles in a reaction cell and suppress the loss of the magnetic particles in a case of suctioning a liquid, and an examination device that makes it possible to suppress the loss of the magnetic particles and to suppress a decrease in the measurement accuracy.

A magnetic collection unit according to the present disclosure is a magnetic collection unit which, during a washing treatment of separating a labeling substance bound to an examination target substance and a labeling substance not bound to the examination target substance in an examination device using magnetic particles in a solid phase in an antigen-antibody reaction, generates a magnetic field in an inside of a reaction cell accommodating a suspension containing the magnetic particles and magnetically collects the magnetic particles in the suspension on an inner wall surface of the reaction cell, the magnetic collection unit comprising:
a magnetic field generation unit that includes two magnets having a length equal to or longer than a distance from a liquid surface of the suspension in the reaction cell to a bottom surface of the reaction cell and generating a magnetic field across a range from the liquid surface to the bottom surface and includes a non-magnetic body, where the two magnets are disposed such that surfaces not having a magnetic pole face each other with the non-magnetic body being sandwiched therebetween, and the two magnets are disposed such that magnetic poles different from each other face the reaction cell; and
a moving mechanism that moves the magnetic field generation unit between a magnetic collection position at which an upper end of each of the magnets is positioned on the liquid surface or above the liquid surface and a retreat position at which the magnetic field does not affect the reaction cell.

It is preferable that the magnet is a neodymium magnet.

The magnet may be an electromagnet.

It is preferable that the magnetic field generation unit includes a shield plate that blocks a magnetic force, at an end part of the two magnets in an arrangement direction.

A plurality of the magnetic field generation units may be disposed in parallel, and it is preferable that the moving mechanism integrally moves the plurality of the magnetic field generation units.

The magnetic collection unit may include a shield plate that blocks a magnetic force in an arrangement direction at an end part of the plurality of the magnetic field generation units that are disposed in parallel.

The moving mechanism may be such as one that moves the magnetic field generation unit in a depth direction from the liquid surface toward the bottom surface in a state in which the upper end of each of the magnets is positioned at the liquid surface or above the liquid surface.

An examination device according to the present disclosure comprises:
a washing treatment unit that includes the magnetic collection unit according to the present disclosure and carries out the washing treatment;
a detection unit that detects light due to the labeling substance; and
a transport mechanism that transports the reaction cell,
in which the washing treatment unit and the detection unit are disposed along a transport direction of the reaction cell.

According to the magnetic collection unit according to the technology of the present disclosure, it is possible to collect magnetic particles in a reaction cell and suppress the loss of the magnetic particles in a case of suctioning a liquid. In addition, according to the examination device according to the technology of the present disclosure, it is possible to suppress the loss of the magnetic particles and to suppress a decrease in the measurement accuracy.

### BRIEF DESCRIPTION OF THE DRAWINGS

Fig. 1 is a schematic view showing an overall configuration of an examination device.
Fig. 2 is a view showing a treatment step in each unit in a treatment unit of the examination device.
Fig. 3 is a view showing a treatment step in each unit in the treatment unit of the examination device.
Fig. 4 is a perspective view showing a main part of a washing treatment unit.
Fig. 5 is a cross-sectional view taken along line V-V of Fig. 4.
Fig. 6 is a view showing a positional relationship between a magnetic field generation unit of a magnetic collection unit and a reaction cell.
Fig. 7A is a view taken in a direction of an arrow VIIA in Fig. 6, and Fig. 7B is a view taken in a direction of an arrow VIIB in Fig. 6.
Fig. 8 is a view showing the washing treatment step (step ST11 to step ST15).
Fig. 9 is a view showing the washing treatment step (step ST16 to step ST21).
Fig. 10A is a top view of a magnetic field generation unit and a reaction cell in a comparative example, and Fig. 10B is a side view of the magnetic field generation unit and the reaction cell in the comparative example.
Fig. 11A is a top view of the reaction cell in a case where magnetic collection is carried out with a magnetic field generation unit, and Fig. 11B is a top view of the reaction cell in a case where magnetic collection is carried out with a magnetic field generation unit in the comparative example.
Fig. 12 is a view showing a modification example of a moving unit in the magnetic collection unit.
Fig. 13 is a view showing a configuration in which a shield plate is provided in the magnetic collection unit.
Fig. 14 is a view showing a washing treatment unit including a magnetic collection unit in which a plurality of magnetic field generation units are juxtaposed in parallel.
Fig. 15 is a view showing a configuration in which a shield plate is provided in a magnetic collection unit in which a plurality of magnetic field generation units are arranged.

### DESCRIPTION OF THE PREFERRED EMBODIMENTS

Hereinafter, an examination device according to an embodiment of the present disclosure will be described with reference to the drawings. In each drawing, the same constitutional elements are represented by the same reference numerals.

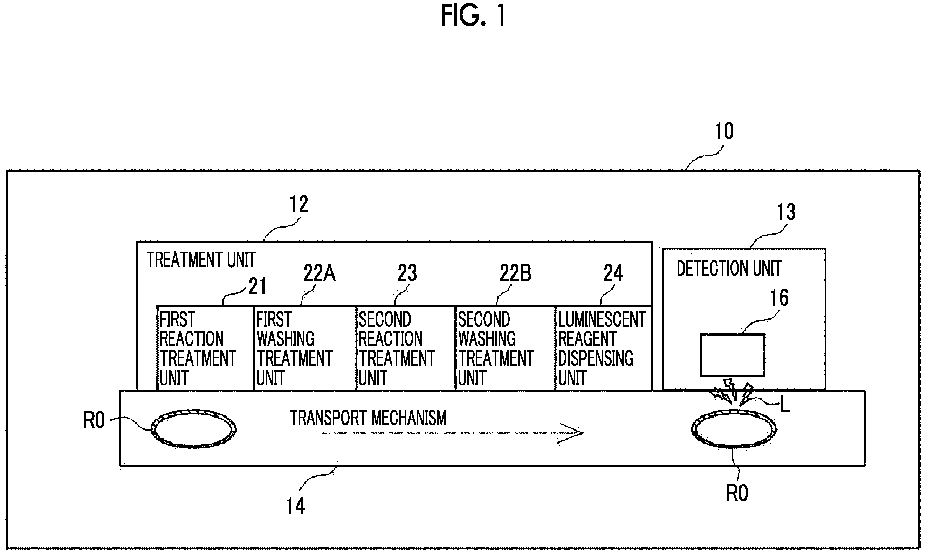

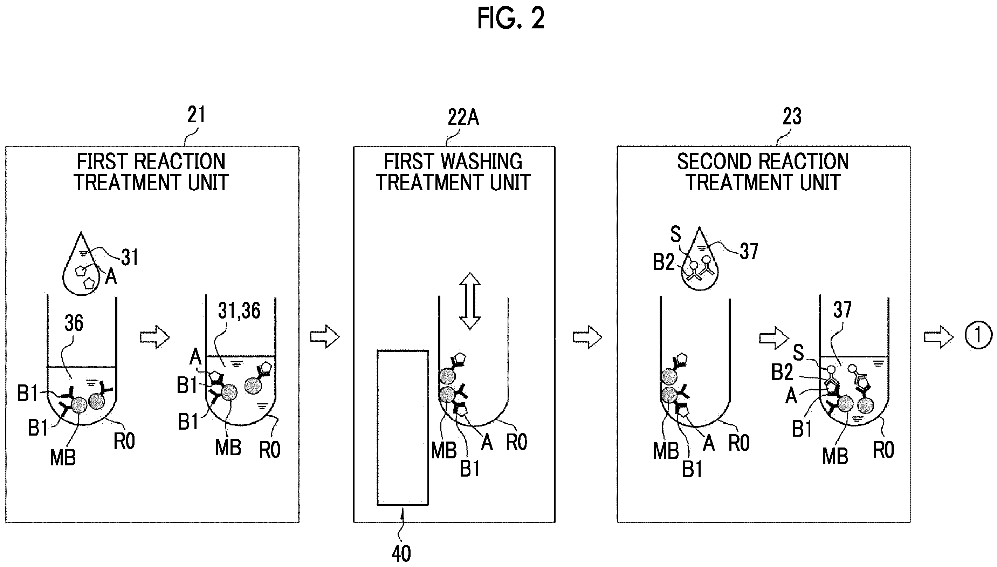

Fig. 1 is a schematic view showing an overall configuration of an examination device 10 according to the embodiment of the present disclosure. The examination device 10 is an immunological analysis device that detects an examination target substance by attaching a labeling substance to an examination target substance in a specimen by using an antigen-antibody reaction, and detecting light due to the labeling substance. The examination device 10 carries out an examination based on, for example, a chemiluminescent enzyme immunoassay method. The examination device 10 uses magnetic particles MB (see Fig. 2) as a solid phase of the antigen-antibody reaction. In the examination device 10, a reaction cell R0 containing the magnetic particles MB as a solid phase is used, and in the reaction cell R0, a treatment of attaching a labeling substance to an examination target substance in a specimen by an antigen-antibody reaction is carried out.

The specimen is, for example, a body fluid such as blood collected from the living body. In a case where the specimen is blood, the specimen may be any of whole blood, blood plasma, serum, or the like. In addition, the examination target substance that can be included in the specimen is an antigen, an antibody, a protein, and a low-molecular-weight compound. It is noted that the specimen is not limited to blood, and may be a substance collected from a living body, such as urine or body fluid.

In a case where the magnetic particles MB that are used as the solid phase have, for example, a spherical shape, the diameter thereof is, for example, 0.1 to 10 µm, preferably 0.1 to 5 µm, and more preferably about 1 to 3 µm. A first binding substance that specifically binds to an examination target substance is attached to the magnetic particles MB.

As shown in Fig. 1, the examination device 10 includes, for example, a treatment unit 12, a detection unit 13, and a transport mechanism 14.

The transport mechanism 14 transports the reaction cell R0 in the examination device 10. The treatment unit 12 and the detection unit 13 are disposed along the transport direction of the reaction cell R0 that is transported by the transport mechanism 14. As a result, the reaction cell R0 transported by the transport mechanism 14 is sequentially transported to the treatment unit 12 and the detection unit 13.

In the detection unit 13, a detection treatment of detecting an examination target substance in the specimen is carried out. The detection unit 13 includes a photodetector 16 such as a photomultiplier tube or a photodiode. The photodetector 16 is disposed to face the reaction cell R0 and detects light L due to the labeling substance bound to the examination target substance. In the present example, an enzyme is used as the labeling substance, and chemiluminescence (hereinafter, referred to as chemiluminescence L) generated by a reaction between the enzyme and a luminescent substrate is detected as the light L due to the labeling substance. The photodetector 16 optically detects an examination target substance to which a labeling substance has been attached by receiving the chemiluminescence L. It is noted that the examination device 10 includes a processor that is not shown in the drawing, and the photodetector 16 outputs a light-receiving signal corresponding to the amount of light received, to the processor. The processor detects whether or not the specimen contains the examination target substance and the concentration thereof based on the light-receiving signal output from the photodetector 16.

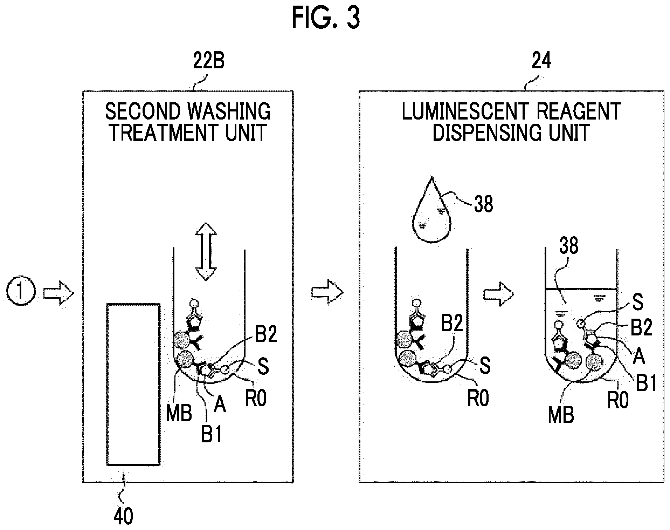

In the treatment unit 12, the treatment of attaching a labeling substance to the examination target substance is carried out by the antigen-antibody reaction described above. In the treatment unit 12, a first reaction treatment unit 21, a first washing treatment unit 22A, a second reaction treatment unit 23, a second washing treatment unit 22B, and a luminescent reagent dispensing unit 24 are disposed in this order from the upstream side in the transport direction along the transport direction of the reaction cell R0. Fig. 2 and Fig. 3 show views schematically showing a treatment that is carried out in each unit of the treatment unit 12.

As shown in Fig. 2, in the first reaction treatment unit 21, a specimen 31 is dispensed into the reaction cell R0, and the specimen 31 is mixed with a reagent 36 containing the magnetic particles MB to which a first binding substance B1 is fixed in the reaction cell R0. The first binding substance B1 is a substance that specifically binds to an examination target substance A, and in a case where the examination target substance A is present in the specimen 31, a first reaction in which the examination target substance A binds to the first binding substance B1 occurs. By this first reaction, an immune complex of the examination target substance A and the first binding substance B1 is formed, and the examination target substance A is captured by the magnetic particles MB through the first binding substance B1. It is noted that the first reaction is promoted by sufficiently dispersing the magnetic particles MB in the reagent 36 and the specimen 31.

As shown in Fig. 2, in the first washing treatment unit 22A, a washing treatment of carrying out B/F separation in which a reacted substance and an unreacted substance in a mixed liquid obtained from the reagent 36 containing the magnetic particles MB and the specimen 31 are separated is carried out. The first washing treatment unit 22A includes a magnetic collection unit 40 described later, and the magnetic collection unit 40 is used during the B/F separation. In the drawing, a bidirectional arrow schematically shows a state in which a liquid is taken in or taken out from the reaction cell R0. Details of the washing treatment will be described later.

As shown in Fig. 2, in the second reaction treatment unit 23, a labeling reagent 37 is dispensed into the reaction cell R0, and in the reaction cell R0, the labeling reagent 37 containing the second binding substance B2 to which a labeling substance S has been attached is mixed with the magnetic particles MB. The second binding substance B2 is a substance that specifically binds to the examination target substance A, and in a case where the examination target substance A is captured by the magnetic particles MB, a second reaction in which the second binding substance B2 binds to the examination target substance A occurs. By this second reaction, a sandwich-type immune complex in which the examination target substance A is sandwiched between the first binding substance B1 and the second binding substance B2 is formed, and the second binding substance B2 is captured by the magnetic particles MB through the examination target substance A and the first binding substance B1. It is noted that the second reaction is promoted by sufficiently dispersing the magnetic particles MB in the labeling reagent 37.

As shown in Fig. 3, in the second washing treatment unit 22B, a washing treatment of carrying out B/F separation in which a reacted substance and an unreacted substance in a mixed liquid of the magnetic particles MB and the labeling reagent 37 are separated is carried out. Similar to the first washing treatment unit 22A, the second washing treatment unit 22B includes the magnetic collection unit 40, and the magnetic collection unit 40 is used during the B/F separation. The washing treatment method is the same as the washing treatment in the treatment carried out by the first washing treatment unit 22A, and the details thereof will be described later. Even in Fig. 3 similar to in Fig. 2, a bidirectional arrow schematically shows a state in which a liquid is taken in or taken out from the reaction cell R0.

In the luminescent reagent dispensing unit 24, a luminescent reagent 38 is dispensed to the reaction cell R0, and the magnetic particles MB and the luminescent reagent 38 are mixed. The luminescent reagent 38 is a reagent that reacts with the labeling substance S to generate the chemiluminescence L (see Fig. 1). It is noted that the generation of the chemical luminescence L is promoted by sufficiently dispersing the magnetic particles MB in the luminescent reagent 38.

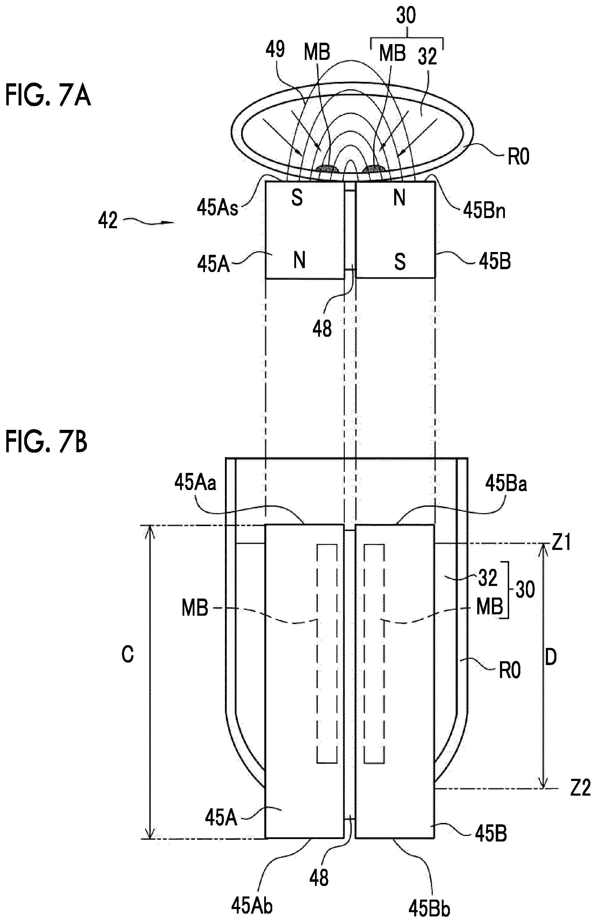

The first washing treatment unit 22A and the second washing treatment unit 22B have substantially the same configuration. Therefore, in a case where they are not particularly required to be distinguished from each other, the configuration and the function thereof will be described as the washing treatment unit 22. All of the mixed liquid of the reagent 36 containing the magnetic particles MB and the specimen 31, the mixed liquid of the magnetic particles MB and the labeling reagent 37, and the mixed liquid of the magnetic particles MB and the luminescent reagent 38 are a suspension 30 (see Figs. 7A and 7B) in which the magnetic particles MB are dispersed in the liquid 32, and in the following description of the washing treatment, the mixed liquids described above are collectively referred to as the suspension 30. In addition, the liquid 32 is a general term for a reagent or the like in which the magnetic particles MB are dispersed.

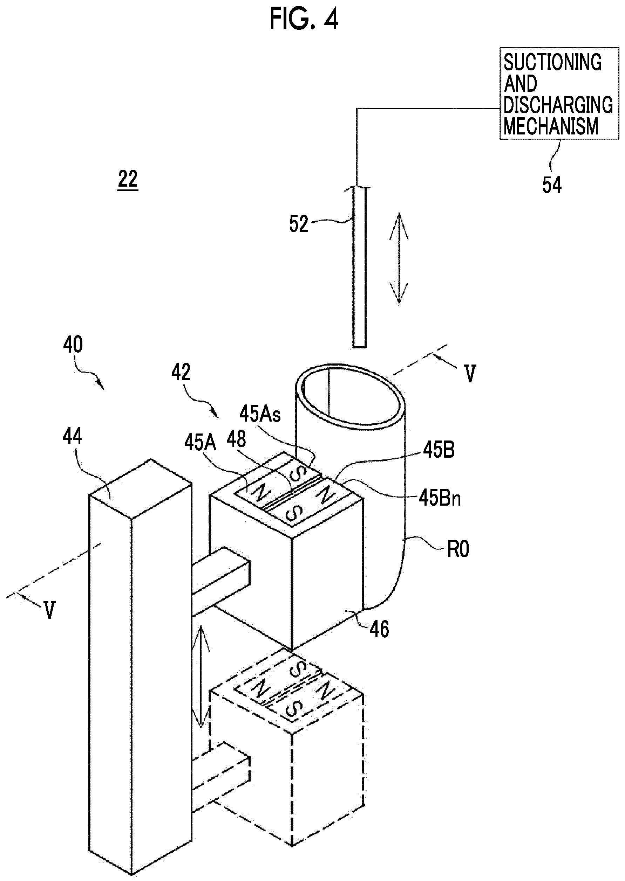

As shown in Fig. 4 as an example, the washing treatment unit 22 includes a suctioning and discharging mechanism 54 that includes the magnetic collection unit 40 that magnetically collects the magnetic particles MB in the reaction cell R0, and a nozzle 52 for suctioning and discharging the washing solution 50 to the reaction cell R0, and a moving mechanism (not shown in the drawing) of the nozzle 52.

The magnetic collection unit 40 generates a magnetic field in the inside of the reaction cell R0 and collects the magnetic particles MB in the suspension 30 on the inner wall surface of the reaction cell R0. The magnetic collection unit 40 includes a magnetic field generation unit 42 and a moving mechanism 44.

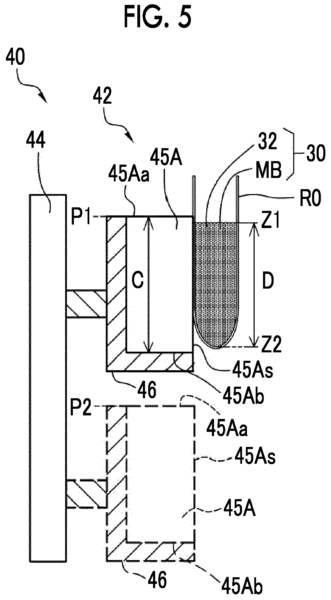

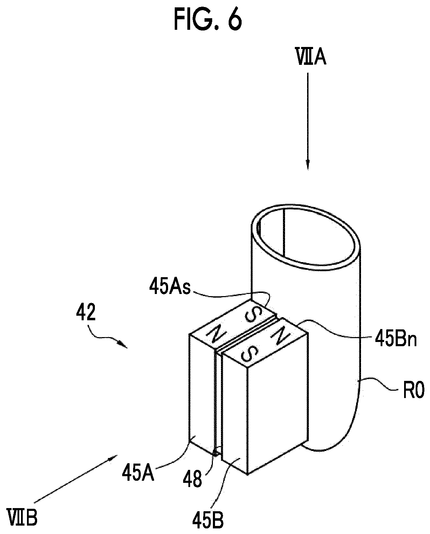

Fig. 5 is a cross-sectional view taken along the line V-V in Fig. 4, and Fig. 6 is a perspective view showing the magnetic field generation unit 42 and the reaction cell R0 by extracting them from Fig. 4. Further, Fig. 7A is a view of the reaction cell R0 and the magnetic field generation unit 42 in Fig. 6, which is taken in a direction of an arrow VIIA as viewed in the direction of the VIIA, and Fig. 7B is a view of the reaction cell R0 and the magnetic field generation unit 42, which is taken in a direction of an arrow VIIB as viewed in the direction of the VIIB.

The magnetic field generation unit 42 includes two magnets 45A and 45B and a non-magnetic body 48 sandwiched between the two magnets 45A and 45B. The two magnets 45A and 45B are disposed such that surfaces having no magnetic poles face each other with the non-magnetic body 48 being sandwiched therebetween. The two magnets 45A and 45B have adjacent magnetic poles different from each other, and the magnetic poles different from each other are disposed to face the reaction cell R0. In the example shown in Fig. 4 to Fig. 7, the magnet 45A is disposed such that the magnetic pole 45As which is an S pole faces the side surface of the reaction cell R0, and the magnet 45B is disposed such that the magnetic pole 45Bn which is an N pole faces the side surface of the reaction cell R0.

As shown in Fig. 5, each of two of the magnet 45A and the magnet 45B has a length C equal to or longer than a distance D from at least a liquid surface Z1 of the suspension 30 in the reaction cell R0 to a bottom surface Z2 of the reaction cell R0. It is noted that the amount of the liquid 32 dispensed into the reaction cell R0 is determined in advance, and the position of the liquid surface Z1 is already known. As a result, the distance D is already known. The length C of each of the magnet 45A and the magnet 45B may be appropriately set according to the distance D. The length C of each of the magnets 45A and 45B needs only to be equal to or larger than the distance D; however, as shown in Fig. 5 as an example, it is desirable that the length C is larger than the distance D. It is noted that the length C of each of the magnets 45A and 45B is preferably a length equal to or less than 130% of the distance D. It is noted that in the present example, although the length of each of the magnet 45A and the magnet 45B is equal to each other, the length of each of the magnet 45A and the magnet 45B needs only to be equal to or larger than the distance D, and the length of each of the magnets 45A and 45B may be different from each other.

In a case of collecting the magnetic particles MB in the suspension 30, as shown in Figs. 7A and 7B, the magnet 45A and the magnet 45B are disposed such that the magnet 45A and the magnet 45B are positioned in a range from the liquid surface Z1 to the bottom surface Z2 of the suspension 30 with the length directions of the magnets 45A and 45B being along the depth direction in the reaction cell R0. That is, during magnetic collection, the magnets 45A and 45B are disposed such that the upper ends 45Aa and 45Ba of the magnets 45A and 45B are positioned at the position of the liquid surface Z1 or positioned above the liquid surface Z1, and the lower ends 45Ab and 45Bb of the magnets 45A and 45B are positioned at the position of the bottom surface Z2 or below the bottom surface Z2. In a case where the magnets 45A and 45B are disposed in this way, the magnets 45A and 45B can generate a magnetic field across a range from the liquid surface Z1 to the bottom surface Z2 in the reaction cell R0.

It is noted that it is preferable that, during magnetic collection, the upper ends 45Aa and 45Ba of the magnets 45A and 45B are positioned above the liquid surface Z1 by, for example, about 1 mm.

The magnets 45A and 45B are a permanent magnet or an electromagnet. In the present example, an example in which the magnets 45A and 45B are a permanent magnet is shown. As the permanent magnet, a neodymium magnet having a large magnetic force is particularly preferable.

The non-magnetic body 48 has a flat plate shape as an example. It is suitable that the non-magnetic body 48 is, for example, an aluminum plate consisting of aluminum.

The moving mechanism 44 moves the magnetic field generation unit 42 in a depth direction (vertical direction in the drawing) from the liquid surface Z1 toward the bottom surface Z2 in a state in which the upper ends 45Aa and 45Ba of the magnets 45A and 45B are positioned at the position of the liquid surface Z1 or above the liquid surface Z1. In the present example, the moving mechanism 44 makes the magnets 45A and 45B movable between a first position (magnetic collection position) where the upper ends 45Aa and 45Ba of the magnets 45A and 45B are at a position P1 above the liquid surface Z1 and a second position where the upper ends 45Aa and 45Ba of the magnets 45A and 45B are at a position P2 below the bottom surface Z2 of the reaction cell R0. The second position is a retreat position where the magnetic field from the magnets 45A and 45B has little effect on the inside of the reaction cell R0.

The magnets 45A and 45B and the non-magnetic body 48 are supported by the support unit 46, and the moving mechanism 44 moves the magnetic field generation unit 42 together with the support unit 46. The moving mechanism 44 is composed of, for example, a linear actuator and the like.

As shown in Fig. 6, the magnets 45A and 45B are disposed such that the magnetic poles 45As and 45Bn of the magnets 45A and 45B abut on the side surface of the reaction cell R0. In addition, the moving mechanism 44 is configured to be movable in the vertical direction, that is, in the depth direction from the liquid surface Z1 toward the bottom surface Z2 in a state in which the magnetic poles 45As and 45Bn are allowed to abut on the side surface of the reaction cell R0.

As shown in Fig. 7A, a magnetic field indicated by a magnetic force line 49 is generated in the reaction cell R0 by the magnets 45A and 45B. The magnetic particles MB in the suspension 30 in the reaction cell R0 are attracted to the magnets 45A and 45B, moved horizontally in the arrow direction, and magnetically collected on the inner wall surface of the reaction cell R0. As shown in Fig. 7B, the magnet 45A and the magnet 45B are disposed from the liquid surface Z1 to the bottom surface Z2 of the reaction cell R0. Therefore, the magnetic particles MB are magnetically collected in a linear region indicated by a broken line in the drawing along a length direction of each of the magnet 45A and the magnet 45B. That is, the magnetic particles MB are magnetically collected in a linear shape of two lines on the inner wall surface of the reaction cell R0.

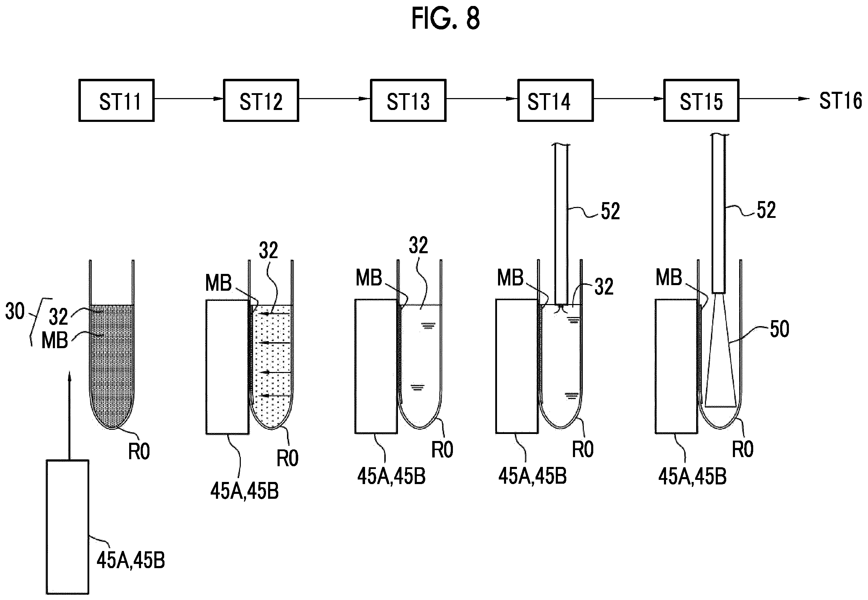

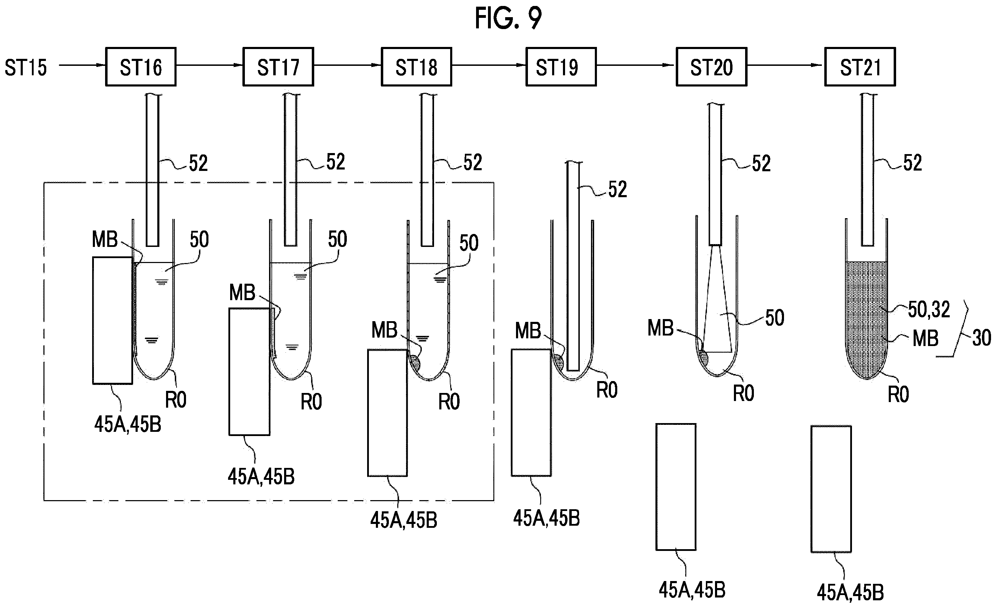

Here, the details of the washing treatment step of carrying out the B/F separation will be described with reference to Fig. 8 and Fig. 9.

In the reaction cell R0 immediately before the start of the washing treatment shown in the step ST11 of Fig. 8, the suspension 30 in which the magnetic particles MB are dispersed in the liquid 32 is accommodated. At this point in time, the magnets 45A and 45B are positioned at a position where the magnetic field is not applied to the reaction cell R0. From this state, the magnets 45A and 45B are moved upward and are disposed on the side surface of the reaction cell R0 as shown in the step ST12. As a result, the magnetic particles MB in the reaction cell R0 are attracted to the magnets 45A and 45B and moved in the arrow direction. Since the magnets 45A and 45B are disposed along the side wall surface of the reaction cell R0 from the liquid surface Z1 of the suspension 30 to the bottom surface Z2, the magnetic particles MB dispersed in the liquid 32 move substantially horizontally so that the movement is in the shortest distance toward the magnets 45A and 45B in the liquid 32.

Therefore, as shown in the step ST13, the magnetic particles MB are magnetically collected in a linear shape along the length directions of the magnets 45A and 45B on the inner wall surface of the reaction cell R0.

Next, as shown in the step ST14, a nozzle 52 for washing is inserted into the reaction cell R0 in a state in which the magnetic particles MB are magnetically collected in a linear shape on the inner wall surface of the reaction cell R0, and the liquid 32 in the reaction cell R0 is suctioned. The nozzle 52 for washing is gradually lowered to the bottom surface side of the reaction cell R0 while suctioning the liquid 32. Thereafter, as shown in the step ST15, the washing solution 50 is discharged from the nozzle 52 for washing in a state in which the nozzle 52 for washing is pulled up. It is noted that the step ST14 and the step ST15 of carrying out suction and discharge in a state in which the magnetic particles MB are magnetically collected may be repeated a plurality of times.

Thereafter, as shown in the step ST16 to the step ST18 of Fig. 9, the magnets 45A and 45B are gradually allowed to move downward along the wall surface of the reaction cell R0 in a state in which the magnetic particles MB are magnetically collected in a linear shape on the inner wall surface of the reaction cell R0. As the magnets 45A and 45B are moved, the magnetic particles MB move downward, and the state of the magnetic particles MB is shifted from a state in which the magnetic particles MB are magnetically collected in a linear shape (see the step ST16) to a state in which the magnetic particles MB are magnetically collected in a dot shape on the inner wall surface close to the bottom surface of the reaction cell R0 (see the step ST18).

As shown in the step ST18, the movement of the magnets 45A and 45B are stopped in a state in which the magnetic particles MB are magnetically collected in a dot shape, and then, as shown in the step ST19, the washing solution 50 in the reaction cell R0 is suctioned by the nozzle 52 for washing. In this case, the magnetic particles MB are magnetically collected in a dot shape at a position deviated from the distal end of the nozzle 52 so that the nozzle 52 does not suction the magnetic particles MB.

After the washing solution 50 in the reaction cell R0 is suctioned, the magnets 45A and 45B are moved to the retracting position as shown in the step ST20. As a result, the state becomes such that the magnetic field generated by the magnets 45A and 45B does not affect the inside of the reaction cell R0. Thereafter, the washing solution 50 is discharged from the nozzle 52 into the reaction cell R0. As a result, the magnetic particles MB are dispersed in the washing solution 50 as shown in the step ST21. It is noted that in the step ST21 of Fig. 9, reference numerals 50 and 32 are written together to indicate that, in the present step, the washing solution 50 is the liquid 32 in which the magnetic particles MB are dispersed. As described above, the liquid 32 is a general term for a reagent or the like in which the magnetic particles MB are dispersed, and in the step ST21 of Fig. 9, the washing solution 50 corresponds to the liquid 32.

It is noted that in the washing treatment unit 22, the steps of the step ST11 to the step ST21 described above are repeated a plurality of times, for example, about three times. The B/F separation is carried out by this washing treatment step.

As described above, the magnetic collection unit 40 includes the magnetic field generation unit 42 including the magnets 45A and 45B that have a length C equal to or longer than a distance D from a liquid surface Z1 of the suspension 30 in the reaction cell R0 to a bottom surface Z2 of the reaction cell R0 and generates a magnetic field across a range from the liquid surface Z1 to the bottom surface Z2. Therefore, in a case where the magnets 45A and 45B are disposed such that the magnets 45A and 45B are positioned in a range from the liquid surface Z1 to the bottom surface Z2 of the suspension 30 with the length direction being along the depth direction in the reaction cell R0, the magnets 45A and 45B can generate a magnetic field at the same time in a range from the liquid surface Z1 to the bottom surface Z2 in the reaction cell R0. In a case where a magnet having a length shorter than the distance D between the liquid surface Z1 and the bottom surface Z2 is used, at least a part of the magnetic particles MB in the suspension 30 move in an oblique direction intersecting the horizontal direction, thereby reaching the inner wall surface. On the other hand, in the present embodiment, since substantially all of the magnetic particles MB in the suspension 30 move in a substantially horizontal direction and are magnetically collected in the reaction cell R0, the magnetic particles MB reach the inner wall surface in the shortest distance. As a result, the magnetic collection of the magnetic particles MB can be carried out very quickly.

In addition, the magnetic collection unit 40 includes the two magnets 45A and 45B, and the non-magnetic body 48 that is provided therebetween. Therefore, the magnetic field intensity can be improved, and the magnetic collection force can be improved. As shown by the magnetic force line 49 in Fig. 7A, by causing the magnetic pole 45As and the magnetic pole 45Bn different from each other to be adjacent to each other with the non-magnetic body 48 being sandwiched therebetween, it is possible to form a portion in a linear shape of two lines, which has a high magnetic field intensity. Therefore, the magnetic particles MB can be magnetically collected in a linear shape of two lines. The magnetic particles MB can be magnetically collected quickly as compared with a case where only one magnet is provided.

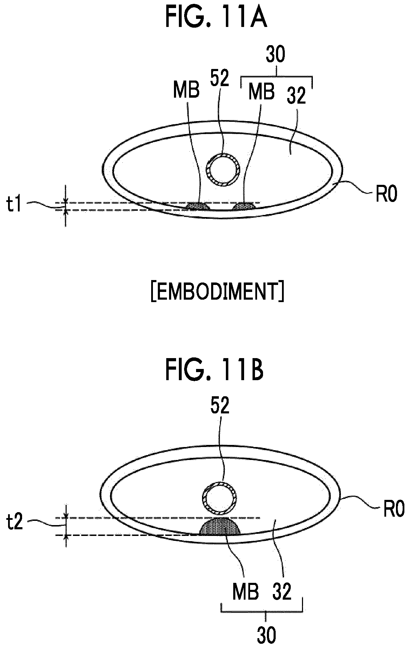

Further, the magnetic collection unit 40 magnetically collects the magnetic particles MB in a linear shape of two lines, whereby the thickness t1 of the aggregate of the magnetic particles MB that have been magnetically collected can be made small (see Fig. 11A), and the loss of the magnetic particles MB during the suction of the liquid 32 by the nozzle 52 can be suppressed.

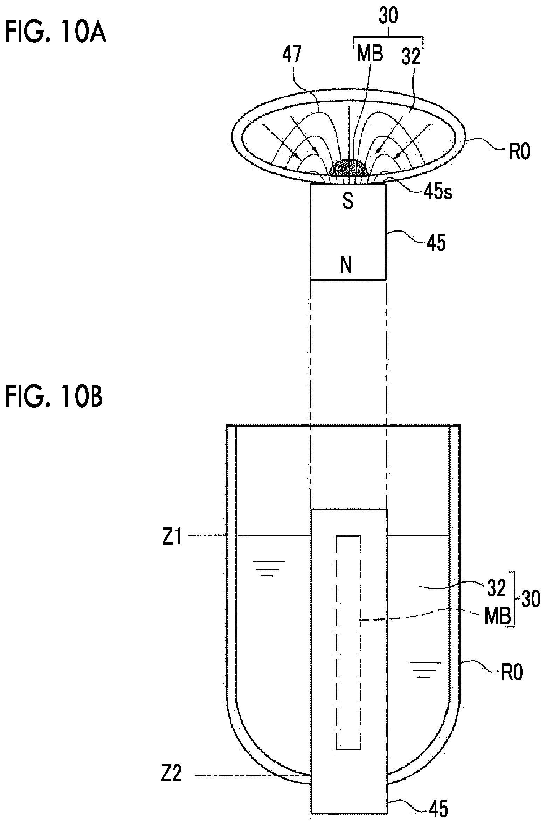

Figs. 10A and 10B are views for describing a magnetic collection state in a case where the magnetic particles MB in the reaction cell R0 are magnetically collected by a magnetic field generation unit composed of only one magnet 45 having the same shape as the magnets 45A and 45B as a comparative example of the magnetic collection unit 40. Fig. 10A is a top view corresponding to Fig. 7A, and Fig. 10B is a side view corresponding to Fig. 7B.

As shown in Fig. 10A, during magnetic collection, for example, the magnet 45 is disposed such that one magnetic pole (here, the S pole) 45s is allowed to abut on the side surface of the reaction cell R0. A magnetic field indicated by a magnetic force line 47 is generated in the reaction cell R0 by the magnet 45. The magnetic particles MB in the suspension 30 in the reaction cell R0 are attracted to the magnet 45, moved in the arrow direction, and magnetically collected on the inner wall surface of the reaction cell R0. As shown in Fig. 10B, the magnet 45 is disposed from the liquid surface Z1 to the bottom surface Z2 of the reaction cell R0. Therefore, the magnetic particles MB are magnetically collected in a linear region indicated by a broken line in the drawing along a length direction of the magnet 45. In the embodiment shown in Figs. 7A and 7B, the magnetic particles MB are magnetically collected in a linear shape of two lines; however, in the comparative example shown in Figs. 10A and 10B, since there is only one magnet 45, the magnetic particles MB are magnetically collected in a linear shape of one line in the example shown in Fig. 10B.

Fig. 11A shows a top view showing a state in which the nozzle 52 is inserted into the reaction cell R0 in which the magnetic particles MB are magnetically collected in a linear shape of two lines shown in Fig. 7A. Fig. 11B is a top view showing a state in which the nozzle 52 is inserted into the reaction cell R0 in which the magnetic particles MB are magnetically collected in a linear shape of one line shown in Fig. 10A. In Fig. 11A and Fig. 11B, the nozzle 52 is shown in a cross section.

The thickness t1 of the aggregate of the magnetic particles MB that have been magnetically collected in a linear shape of two lines in the reaction cell R0 shown in Fig. 11A is smaller than the thickness t2 of the aggregate of the magnetic particles MB that have been magnetically collected in a linear shape of one line in the reaction cell R0 shown in Fig. 11B. Here, the thicknesses t1 and t2 of the aggregate of the magnetic particles MB are distances between the inner wall surface corresponding to the side surface of the reaction cell R0 where the magnets 45A and 45B or the magnet 45 is allowed to abut on and the position of the aggregate of the magnetic particles MB closest to the central side of the reaction cell R0. It is noted that in a case where the length of the magnet 45 is shorter than the distance D from the liquid surface Z1 to the bottom surface Z2 in the comparative example, the thickness of the aggregate of the magnetic particles MB is further larger than t2. In a case where the nozzle 52 is inserted into the reaction cell R0 in order to suction the liquid 32 in a state in which the magnetic particles MB are magnetically collected, the thinner the thickness of the aggregate of the magnetic particles MB, the more the distal end of the nozzle 52 can be separated from the magnetic particles MB.

As shown in Fig. 11A, in the example of the embodiment, the nozzle 52 can be inserted into the reaction cell R0 in a state in which a certain distance is ensured from the aggregate of the magnetic particles MB. On the other hand, as shown in Fig. 11B, in the comparative example, the distance between the nozzle 52 and the aggregate of the magnetic particles MB is shortened. As the distance between the nozzle 52 and the aggregate of the magnetic particles MB becomes shorter, the distal end of the nozzle is more likely to come into contact with the magnetic particles MB during the insertion of the nozzle 52. In a case where the magnetic particles MB come into contact with the distal end of the nozzle 52 and are attached to the distal end of the nozzle 52 during the insertion of the nozzle 52 and/or in a case where the distance from the distal end of the nozzle 52 to the aggregate of the magnetic particles MB is close during the suction, the magnetic particles MB are easily suctioned during the suction of the liquid 32 by the nozzle 52. As shown in Fig. 11A, in the example of the embodiment, since the thickness of the aggregate of the magnetic particles MB is small (t1 < t2) as compared with the comparative example shown in Fig. 11B, it is possible to suppress the contact with the aggregate of the magnetic particles MB in a case where the nozzle 52 is inserted into the reaction cell R0. In addition, it is possible to further separate the distance between the distal end of the nozzle 52 and the aggregate of the magnetic particles MB during suction. In addition, as a result, in the example of the embodiment, the suction of the magnetic particles MB can be suppressed as compared with the comparative example. It is noted that since the loss of the magnetic particles MB due to the suction of the magnetic particles MB leads to a decrease in measurement accuracy, the decrease in measurement accuracy can be suppressed by suppressing the suction of the magnetic particles MB.

In addition, the magnetic collection unit 40 of the present embodiment includes the moving mechanism 44 and moves the magnetic field generation unit 42 (here, the magnets 45A and 45B) in a depth direction from the liquid surface Z1 toward the bottom surface Z2 in a state in which the upper ends 45Aa and 45Ba of the magnets 45A and 45B are positioned at the liquid surface Z1 or above the liquid surface Z1. As a result, the magnetic particles MB that have been magnetically collected in a linear shape in the length directions of the magnets 45A and 45B can be magnetically collected in a dot shape in the vicinity of the bottom surface of the reaction cell R0. By changing the magnetic collection form from a linear shape to a dot shape in this way, suctioning the liquid 32, and then discharging the washing solution 50 from the nozzle 52 such that the washing solution 50 comes into contact with the magnetic particles MB that have been magnetically collected in a dot shape, the dispersibility of the magnetic particles MB can be improved in a case where the magnetic particles MB are redispersed in the liquid. In a case where the dispersibility of the magnetic particles MB is increased, the washability of the magnetic particles MB is improved, and the B/F separation can be carried out with higher accuracy. In the examination device 10, by using such a magnetic collection unit 40, it is possible to reduce noise associated with the effect of improving the B/F separation accuracy, and, in the long run, it is possible to suppress the occurrence of a measurement error and obtain a highly accurate measurement result. Even in a case where the magnetic particles MB are not magnetically collected in a dot shape, it is possible to increase the dispersibility by repeating the suction and discharge of the washing solution 50 after the washing solution 50 is discharged. However, there is such an effect that the time until sufficient dispersibility is ensured can be shortened by discharging the washing solution 50 so that the washing solution 50 comes into contact with the magnetic particles MB that have been magnetically collected in a dot shape.

In the magnetic collection unit 40 described above, the magnetic field generation unit 42 is disposed in a state in which the magnetic poles 45As and 45Bn of the magnets 45A and 45B are allowed to abut on a side surface of the reaction cell R0, and the moving mechanism 44 moves the magnetic field generation unit 42 in the depth direction in a state where the magnetic pole 45As of the magnet 45A and the magnetic pole 45Bn of the magnet 45B are allowed to abut on the side surface of the reaction cell R0. The magnetic field generation unit 42 may be disposed such that the magnetic pole 45As of the magnet 45A and the magnetic pole 45Bn of the magnet 45B are brought close to the side surface of the reaction cell R0 without abutting on the side surface. However, by causing the magnetic poles 45As and 45Bn to abut on the reaction cell R0, the intensity of the magnetic field generated in the reaction cell R0 can be increased, the magnetic collection effect can be increased, and the magnetic collection can be carried out more quickly. In addition, even in a case where the magnetic field generation unit 42 is moved, the magnetic field generation unit 42 is moved in a state in which the magnetic poles 45As and 45Bn are allowed to abut on the side surface of the reaction cell R0, whereby the magnetic particles MB smoothly follow the magnets 45A and 45B in a case where the magnetic collection form is changed from a linear shape to a dot shape.

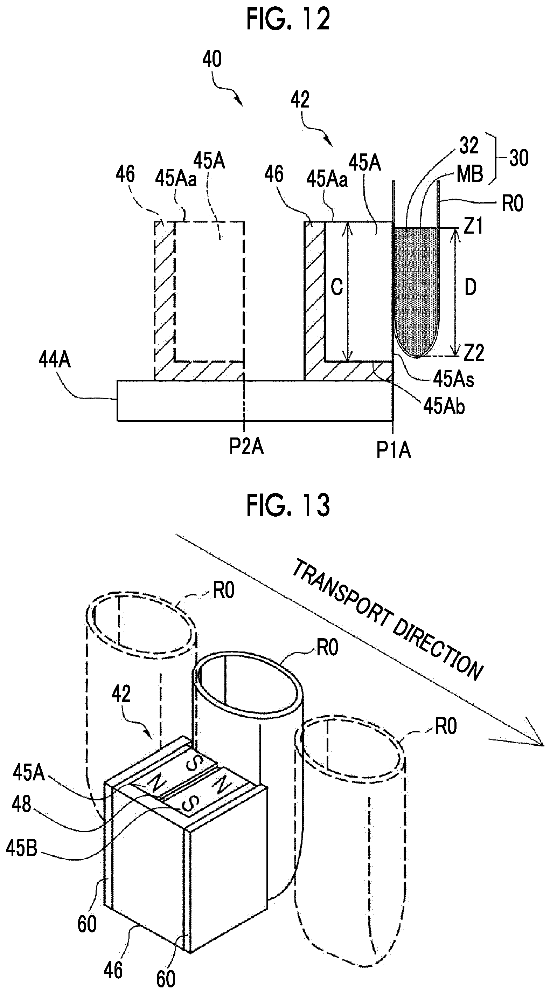

In the magnetic collection unit 40 shown in Fig. 4, the moving mechanism 44 is configured to collect the magnetic particles MB in a linear shape, change the magnetic collection form to a dot shape, and further, be movable only in the uniaxial direction (vertical direction) to the retreat position. However, the form of the moving mechanism 44 is not limited thereto, and the moving mechanism 44 may be configured to be movable in the vertical direction in a range in which the magnetic collection form of the magnetic particles MB can be changed from a linear shape to a dot shape, and further, may be configured to be movable in a direction away from the reaction cell R0 in the horizontal direction. In this case, the retreat position may be such a position away from the reaction cell R0 in the horizontal direction that the magnetic field from the magnetic field generation unit 42 does not affect the reaction cell R0. In addition, the moving mechanism of the present disclosure does not necessarily have a configuration in which the magnetic collection form can be changed into a dot shape as long as the magnetic particles MB can be magnetically collected in a linear shape of two lines. For example, as in a modification example shown in Fig. 12, the moving mechanism 44A may be a moving mechanism that moves the magnetic field generation unit 42 horizontally between a first position (magnetic collection position) P1A at which the magnets 45A and 45B are allowed to abut on the side surface of the reaction cell R0 and a second position (retreat position) P2A separated from the reaction cell R0 in the horizontal direction.

By using a strong magnet such as a neodymium magnet as the magnets 45A and 45B, it is possible to reliably generate a magnetic field in the reaction cell R0 and enhance the magnetic collection function of the magnetic particles MB. The magnet provided in the magnetic field generation unit 42 is not limited to a permanent magnet, and an electromagnet may be used. In a case where an electromagnet is used, the on and off of the magnetic field generated in the reaction cell R0 can be switched by the on and off of the current, Therefore, it is not necessary to move the magnet to the retreat position in a case where the current is turned off after the magnet is moved to change the magnetic collection form from a linear shape to a dot shape and the liquid is suctioned. On the other hand, in a case where a permanent magnet is used, electric power for generating a magnetic field is not required, and it is possible to simplify a wiring line and the like.

It is noted that, as shown in Fig. 13, it is preferable that the magnetic field generation unit 42 includes a shield plate 60 that blocks a magnetic force, at an end part of the two magnets in an arrangement direction 45A and 45B. In the example shown in Fig. 13, the shield plate 60 is provided outside the support unit 46. It is noted that the support unit 46 may be composed of a shielding material that blocks a magnetic force instead of including the shield plate 60. That is, the support unit 46 may also serve as the shield plate 60.

In the examination device 10 (see Fig. 1), the reaction cell R0 is transported in a transport direction indicated by an arrow in Fig. 13 by the transport mechanism 14. In this case, the transport mechanism 14 can transport a plurality of reaction cells R0 at the same time, and as shown in Fig. 13, the plurality of reaction cells R0 are transported in a state of being adjacent to each other. As shown in Fig. 1, the respective treatment units are disposed in order along the transport direction. Therefore, in a case where the magnetic field generation unit 42 generates, for B/F separation, a magnetic field shown by a solid line in Fig. 13 with respect to the reaction cell R0, the reaction cells R0 disposed adjacent to each other, which are indicated by a broken line may be subjected to a treatment that is required to disperse the magnetic particles MB in the liquid. The treatment that is required to disperse the magnetic particles MB in the liquid is, for example, a first reaction treatment, a second reaction treatment, or a luminescent reagent dispensing treatment. In a case where the magnetic field generated by the magnetic field generation unit 42 affects the reaction cell R0 that has been subjected to the treatment required to disperse the magnetic particles MB in the liquid, it is also conceivable that the deviation may occur in the magnetic particles MB in the reaction cell R0, and the dispersibility may be lowered. On the other hand, by providing the shield plate 60, it is possible to suppress the generation of a magnetic field in the reaction cell R0 adjacent to the reaction cell R0 that is a target in which a magnetic field is generated. That is, by providing the shield plate 60, it is possible to reduce adverse effects such as inhibition of the dispersibility of the magnetic particles MB in the reaction cell R0 adjacent to the reaction cell R0 that is a target in which a magnetic field is generated.

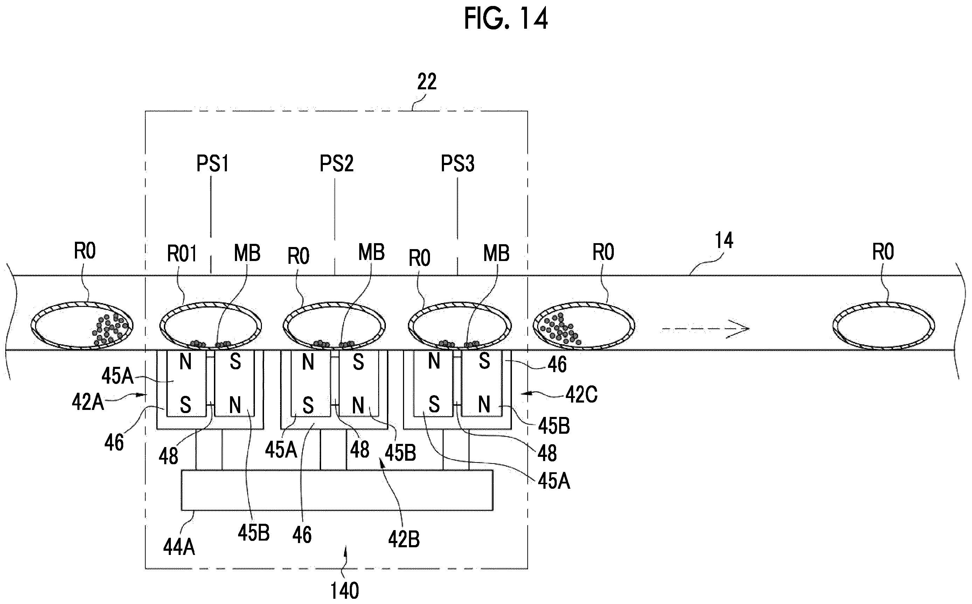

Further, in the magnetic collection unit according to the present disclosure, a plurality of the magnetic field generation units 42 may be disposed in parallel so that a magnetic field can be generated at the same time for the plurality of reaction cells R0. Fig. 14 shows a magnetic collection unit 140 including a plurality of magnetic field generation units 42.

In the magnetic collection unit 140 shown in Fig. 14, three magnetic field generation units 42 are juxtaposed in parallel. In Fig. 14, for convenience, detailed reference numerals A to C are given for the reference numerals of the magnetic field generation units 42A, 42B, and 42C, and the magnetic field generation unit 42. In a case where they are not required to be distinguished from each other, they are simply referred to as the magnetic field generation unit 42.

The three magnetic field generation units 42 are arranged adjacent to each other. The magnets adjacent to each other between the magnetic field generation units 42 are arranged to have magnetic poles different from each other. For example, in Fig. 14, in the magnetic field generation unit 42A and the magnetic field generation unit 42B which are disposed adjacent to each other, the magnet 45B disposed on the magnetic field generation unit 42B side of the magnetic field generation unit 42A and the magnet 45A disposed on the magnetic field generation unit 42A side of the magnetic field generation unit 42B are disposed such that the S pole and the N pole are adjacent to each other.

The magnetic field generation units 42 are each supported by the support unit 46, and the moving mechanism 44A is configured such that the three magnetic field generation units 42 are movable together with each of the support units 46. The moving mechanism 44A integrally moves the three magnetic field generation units 42. Similar to the moving mechanism 44 described above, the moving mechanism 44A is composed of, for example, a linear actuator and the like.

In the examination device 10 of Fig. 1, the transport mechanism 14 transports a plurality of reaction cells R0 at the same time, and the plurality of reaction cells R0 are subjected to a treatment in parallel in each treatment unit. As described above, the washing treatment step (step ST11 to Step ST21) described with reference to Fig. 8 and Fig. 9 is repeated, for example, three times. In this case, as shown in Fig. 14, three reaction cells R0 can be subjected to the washing treatment at the same time in a case where the washing treatment unit 22 is provided from the position PS1 to the position PS3, and the washing treatment step can be carried out at each of the position PS1, the position PS2, and the position PS3. In Fig. 14, for convenience of description, a detailed reference numeral 1 is given to one reaction cell R0 present at the position PS1 and is indicated as a reaction cell R01. For example, after subjecting the reaction cell R01 to the first washing treatment at the position PS1, the reaction cell R01 is moved to the position PS2 and subjected to the second washing treatment, and further, the reaction cell R01 is moved to the position PS3 and subjected to the third washing treatment. In this way, in a case where the washing treatment is carried out at each position while sequentially carrying out transporting from the position PS1 to the position PS3, the treatment can be efficiently carried out.

In a case where the magnetic collection unit 140 is used, three magnetic field generation units 42 can be integrally driven and magnetic collection can be carried out by one moving mechanism 44A in a case where three reaction cells R0 transported to three positions PS1 to PS3 are subjected to the washing treatment. In the magnetic collection unit 140, since the three magnetic field generation units 42 are integrally driven by one moving mechanism 44A, the cost can be suppressed as compared with a case where the moving mechanism is individually provided at each of the position PS1 to the position PS3.

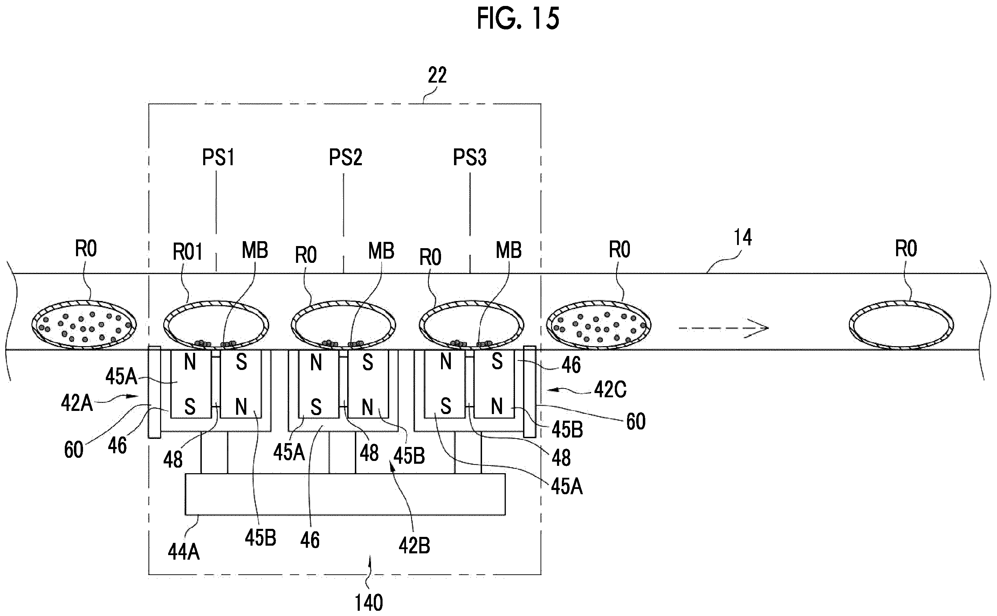

As shown in Fig. 15, it is preferable that the shield plate 60 that blocks a magnetic force in an arrangement direction at an end part of the plurality of the magnetic field generation units 42 that are disposed in parallel is provided in the magnetic collection unit 140 as well. As shown in Fig. 14, in a case where the shield plate 60 is not provided, the magnetic field generated by the magnetic field generation unit 42 may affect the reaction cell R0 positioned outside the washing treatment unit 22, which is disposed adjacent to the reaction cell R0 that is a target in which a magnetic field is generated. In this case, as shown in Fig. 14, the deviation occurs in the magnetic particles MB in the reaction cell R0 disposed adjacent to the reaction cell R0 that is a target in which a magnetic field is generated. On the other hand, as shown in Fig. 15, in a case where the shield plate 60 is provided, it is possible to suppress the influence of the magnetic field generated by the magnetic field generation unit 42 on the reaction cell R0 that is not a target in which a magnetic field is generated. That is, in a case where the shield plate 60 is provided, it is possible to suppress a decrease in the dispersibility of the magnetic particles MB in the liquid in the reaction cell R0 that is not a target in which a magnetic field is generated, that is, in the reaction cell R0 that is positioned outside the washing treatment unit 22.

In regard to the embodiment described above, the following supplementary notes will be further disclosed.

### (Supplementary Note 1)

A magnetic collection unit which, during a washing treatment of separating a labeling substance bound to an examination target substance and a labeling substance not bound to the examination target substance in an examination device using magnetic particles in a solid phase in an antigen-antibody reaction, generates a magnetic field in an inside of a reaction cell accommodating a suspension containing the magnetic particles and magnetically collects the magnetic particles in the suspension on an inner wall surface of the reaction cell, the magnetic collection unit comprising:
a magnetic field generation unit that includes two magnets having a length equal to or longer than a distance from a liquid surface of the suspension in the reaction cell to a bottom surface of the reaction cell and generating a magnetic field across a range from the liquid surface to the bottom surface and includes a non-magnetic body, where the two magnets are disposed such that surfaces not having a magnetic pole face each other with the non-magnetic body being sandwiched therebetween, and the two magnets are disposed such that magnetic poles different from each other face the reaction cell; and
a moving mechanism that moves the magnetic field generation unit between a magnetic collection position at which an upper end of each of the magnets is positioned on the liquid surface or above the liquid surface and a retreat position at which the magnetic field does not affect the reaction cell.

### (Supplementary Note 2)

The magnetic collection unit according to the supplementary note 1, wherein the magnet is a neodymium magnet.

### (Supplementary Note 3)

The magnetic collection unit according to the supplementary note 1, wherein the magnet is an electromagnet.

### (Supplementary Note 4)

The magnetic collection unit according to any one of the supplementary note 1 to the supplementary note 3, wherein the magnetic field generation unit includes a shield plate that blocks a magnetic force, at an end part of the two magnets in an arrangement direction.

### (Supplementary Note 5)

The magnetic collection unit according to any one of the supplementary note 1 to the supplementary note 4,
wherein a plurality of the magnetic field generation units are disposed in parallel, and
the moving mechanism integrally moves the plurality of the magnetic field generation units.

### (Supplementary Note 6)

The magnetic collection unit according to the supplementary note 5, wherein a shield plate that blocks a magnetic force is provided in an arrangement direction at an end part of the plurality of the magnetic field generation units that are disposed in parallel.

### (Supplementary Note 7)

The magnetic collection unit according to any one of the supplementary note 1 to the supplementary note 6, wherein the moving mechanism moves the magnetic field generation unit in a depth direction from the liquid surface toward the bottom surface in a state in which the upper end of each of the magnets is positioned at the liquid surface or above the liquid surface.

### (Supplementary Note 8)

An examination device comprising:
a washing treatment unit that includes the magnetic collection unit according to any one of the supplementary note 1 to the supplementary note 7 and carries out the washing treatment;
a detection unit that detects light due to the labeling substance; and
a transport mechanism that transports the reaction cell,
wherein the washing treatment unit and the detection unit are disposed along a transport direction of the reaction cell.

It is noted that the disclosure of JP2022-175125 filed on October 31, 2022, is incorporated in the present specification in its entirety by reference. All of the documents, the patent applications, and the technical standards described in the present specification are incorporated into the present specification by reference to the same extent as in a case in which each of the documents, the patent applications, and the technical standards are specifically and individually stated to be described by reference.

## Claims

1. A magnetic collection unit which, during a washing treatment of separating a labeling substance bound to an examination target substance and a labeling substance not bound to the examination target substance in an examination device using magnetic particles in a solid phase in an antigen-antibody reaction, generates a magnetic field in an inside of a reaction cell accommodating a suspension containing the magnetic particles and magnetically collects the magnetic particles in the suspension on an inner wall surface of the reaction cell, the magnetic collection unit comprising:
a magnetic field generation unit that includes two magnets having a length equal to or longer than a distance from a liquid surface of the suspension in the reaction cell to a bottom surface of the reaction cell and generating a magnetic field across a range from the liquid surface to the bottom surface and includes a non-magnetic body, where the two magnets are disposed such that surfaces not having a magnetic pole face each other with the non-magnetic body being sandwiched therebetween, and the two magnets are disposed such that magnetic poles different from each other face the reaction cell; and
a moving mechanism that moves the magnetic field generation unit between a magnetic collection position at which an upper end of each of the magnets is positioned on the liquid surface or above the liquid surface and a retreat position at which the magnetic field does not affect the reaction cell.

2. The magnetic collection unit according to claim 1, wherein the magnet is a neodymium magnet.

3. The magnetic collection unit according to claim 1, wherein the magnet is an electromagnet.

4. The magnetic collection unit according to claim 1, wherein the magnetic field generation unit includes a shield plate that blocks a magnetic force, at an end part of the two magnets in an arrangement direction.

5. The magnetic collection unit according to claim 1,
wherein a plurality of the magnetic field generation units are disposed in parallel, and
the moving mechanism integrally moves the plurality of the magnetic field generation units.

6. The magnetic collection unit according to claim 5, wherein a shield plate that blocks a magnetic force is provided in an arrangement direction at an end part of the plurality of the magnetic field generation units that are disposed in parallel.

7. The magnetic collection unit according to claim 1, wherein the moving mechanism moves the magnetic field generation unit in a depth direction from the liquid surface toward the bottom surface in a state in which the upper end of each of the magnets is positioned at the liquid surface or above the liquid surface.

8. An examination device comprising:
a washing treatment unit that includes the magnetic collection unit according to claim 1 and carries out the washing treatment;
a detection unit that detects light due to the labeling substance; and
a transport mechanism that transports the reaction cell,
wherein the washing treatment unit and the detection unit are disposed along a transport direction of the reaction cell.
